# EUROPEAN PATENT APPLICATION

(11) **EP 4 729 007 A1**
(43) Date of publication of application: **22.04.2026**
(21) Application number: 23942252.0
(22) Date of filing: 19.06.2023
(51) Int. Cl.: A61B 17/86, A61B 17/88

(54) **INTERNAL FIXATION INSTRUMENT AND TREATMENT INSTRUMENT**

(71) Applicant: Spine Chronicle Japan Co., Ltd., Kanazawa-city, Ishikawa 920-0848 (JP)
(72) Inventor: YONEZAWA, Noritaka, Kanazawa-city, Ishikawa 920-0848 (JP)
(74) Representative: Plougmann Vingtoft a/s
(86) International application number: PCT/JP2023/022593
(87) International publication number: WO 2024/261813

(57) **Abstract**

An internal fixation device that is inserted through the pedicle into bone cement pre-filled into the vertebral body, the device being easy-to-remove, but exhibiting internal fixation performance equivalent to that of a screw is provided. An internal fixation device to be inserted through a pedicle into bone cement filled into a vertebral body, the device comprising a columnar shaft and a connection at a distal end of the shaft that can be connected to a screwdriver, and the shaft has helical grooves on a proximal side. More suitably, the internal fixation device is provided with threads on the distal side of the shaft, the threads and the grooves formed to have the same lead.

## Description

### Technical Field

The present invention relates to a cylindrical internal fixation device for medical use and a treatment device for operating the same, and is particularly suitable for use in internal fixation surgery of an affected vertebral body.

### Background Art

Vertebroplasty and spinal fusion are known treatments for compression fractures of the spine. The spine consists of stacked vertebral bodies and their supporting vertebral arches, pedicles, and articular processes. The vertebral body is a cylindrical bone which is supported by two pedicles on either side extending from the vertebral arch. The vertebral bodies sandwich the nucleus pulposus and the annulus fibrosus surrounds it. The vertebral arches connect vertically via the upper and lower articular processes and support the vertebral body via the pedicles, forming the spine. A compression fracture is a condition in which the vertebral body is crushed and damaged by vertical compression. Vertebroplasty is a surgical procedure to reconstruct the crushed vertebral body, for example, by filling the vertebra with medical cement. Spinal fusion is a technique to fix the crushed vertebral body to undamaged vertebral bodies above and below the crushed one using instruments. Screws are threaded into the crushed vertebral body and uncrushed vertebral bodies above and below the crushed one from the pedicles; and heads of the screws are fixed to each other via a vertical rod connecting them. Vertebroplasty is employed when the conditions are mild, but spinal fusion is required when symptoms are severe.

Various screws have been proposed for use in these treatments.

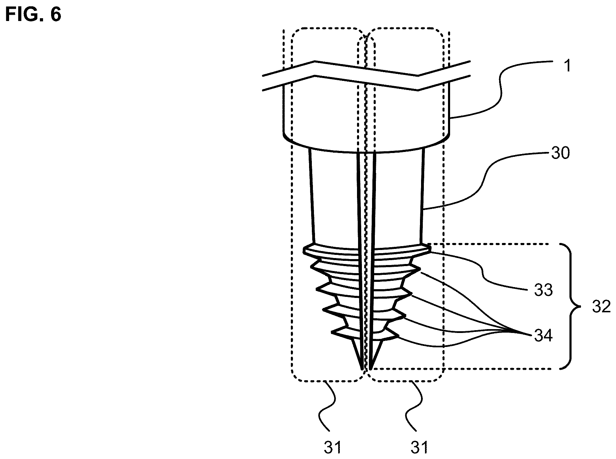

PTL 1 discloses a medical screw provided with a longitudinal through hole along its central axis and a lateral opening communicating with the through hole. An injection instrument such as a syringe is attached to a head of the screw, and bone cement and/or biologically active substances is injected into surrounding bones through the through hole on the rough surface to reinforce the surrounding bones ([0053]-[0057], FIGS. 6-8).

PTL 2 discloses a medical screw that is said to be easily removed from a fracture site it is threaded in. The disclosed is a medical screw with a hollow section extending from a head to a tip along a central axis. The screw has a threaded side surface and is screwed into a bone. The screw is provided with a reverse thread (i.e., a reverse internal thread) near an inside tip of the hollow section, and can be pulled out by using a pulling-out operation jig equipped with a tip section that engages with the reverse internal thread. More specifically, the pulling-out operation jig is inserted through the hollow section, engaging the tip section with the reverse internal thread of the screw for integration, and the pulling-out operation jig is rotated to remove the screw out of the body.

### CITATION LIST

### Patent Document

PTL 1: U.S. Patent Application Publication 2011/0040337A1
PTL 2: Japanese Patent Application Laid-Open No. 2016-209295

### SUMMARY OF THE INVENTION

### Problems to be Solved by the Invention

The present inventor found the problem of insufficient development of medical devices and screws suitable for the combined vertebroplasty and pedicle formation as described above, invented a treatment device to solve this problem, and filed an international application as PCT/JP2022/036147. The applied invention is a treatment device that is integrated with a screw having a through hole along a central axis and threads for tapping on a side surface thereof. The device is configured such that bone cement can be injected into the affected area through the through hole of the screw with a screwdriver connected to a head of the screw, and then the screw can be screwed into the bone cement. Since the screwdriver can be operated without the need to reattach the screwdriver after the injection of the bone cement, the screw can be screwed quickly before the bone cement hardens.

The inventor has realized that if the need to remove the screw arose after the bone cement had hardened, there is risk that the screw would be too tightly engaged in the bone cement and could not be successfully removed. Generally, the need to remove the screw after surgery is rarely considered, but the inventor believes that allowing the screw to be removed would contribute to the treatment of postoperative complications, such as infection of the affected area, from which cannot be entirely free after surgery.

Conventional screws as in PTL 1, on the contrary, are not designed to be screwed into pre-injected bone cement; cement and/or other materials is injected around the screw through an opening in a side wall to reinforce the surrounding bone after the screw is screwed in. Therefore, it is essentially bone that engages with the screw threads, and it is believed that the screw can be pulled out relatively easily by reversing the screw.

If pulling out is still expected to be difficult, a screw and a pulling-out operation jig that is designed for pulling out is used, as described in PTL 2.

The inventor has examined more deeply the risk about the danger that the screw would be too tightly engaged in the bone cement and could not be successfully removed, and has found that such danger is manifested when the bond due to the screw's engagement with the bone cement is stronger than the bond between the bone cement and the surrounding bone and other affected tissue. The inventor has noticed that in that case using a jig for pulling out as described in PTL 2 does not solve the problem. Although a jig for pulling out as described in PTL 2 can strongly connect the screw to a screwdriver and can transmit strong force to the screwdriver, which aids in pulling out when the surrounding area is affected tissue such as the patient's bone, if the bond between the bone cement and the screw is stronger than the bond between the bone cement and the surrounding bone or other affected tissue, the bone cement might separate from the surrounding affected tissue instead of the screw detaching from the bone cement, which will not aid in removal.

An object of the present invention is to provide an internal fixation device that is inserted through the pedicle into bone cement pre-filled into the vertebral body, the device being easy-to-remove, but exhibiting internal fixation performance equivalent to that of a screw.

Means for solving these problems will be described below, but other issues and novel features will become apparent from the description herein and the accompanying drawings.

### Means for Solving Problems

According to one embodiment of the present invention, a means for solving the problems is as follows.

That is, an internal fixation device to be inserted through a pedicle into bone cement filled into a vertebral body, the device including a cylindrical shaft and a connection at a distal end of the shaft that can be connected to a screwdriver, and the shaft has helical grooves on a proximal side.

The term "proximal" herein is a medical term referring to the side closer to, and "distal" refers to the side farther away the centerline of a patient's body. Bone cement is medical cement composed mainly of, for example, calcium phosphate and polymethylmethacrylate, and hardens over time.

### Effect of the Invention

The effect to be produced by the above-described embodiment will be briefly described below.

That is, an internal fixation device that is inserted through the pedicle into bone cement pre-filled into the vertebral body can be provided, the device being easy-to-remove, but exhibiting internal fixation performance equivalent to that of a screw.

### BRIEF DESCRIPTION OF THE DRAWINGS

FIG. 1 is a top view, a front view and a bottom view of an example configuration of an internal fixation device according to the present invention.
FIG. 2 is an explanatory drawing overviewing an example use of the internal fixation device according to the present invention.
FIG. 3 is an explanatory drawing illustrating a solution principle of the present invention.
FIG. 4 is an explanatory drawing showing a procedure of an example use of the internal fixation device according to the present invention.
FIG. 5 is an explanatory drawing schematically illustrating a cross-sectional structure to show an example configuration of a treatment device as a second embodiment of the present invention.
FIG. 6 is an explanatory drawing overviewing an example configuration of a tip section of an inner cylinder.
FIG. 7 is an explanatory diagram schematically illustrating a cross-sectional structure to show an example configuration of the tip section of the inner cylinder.
FIG. 8 is an explanatory drawing schematically illustrating a treatment procedure (first half) using a treatment device according to the present invention.
FIG. 9 is an explanatory drawing schematically illustrating the treatment procedure (second half) using the treatment device according to the present invention.

### DETAILED DESCRIPTION OF THE INVENTION

### 1. Outline of Embodiment

First, an outline of a representative embodiment disclosed in the present application will be given. Reference signs in the drawings that are referred to in parentheses in the outline description of representative embodiment are merely illustrative of what is included in the concept of the components to which they refer.

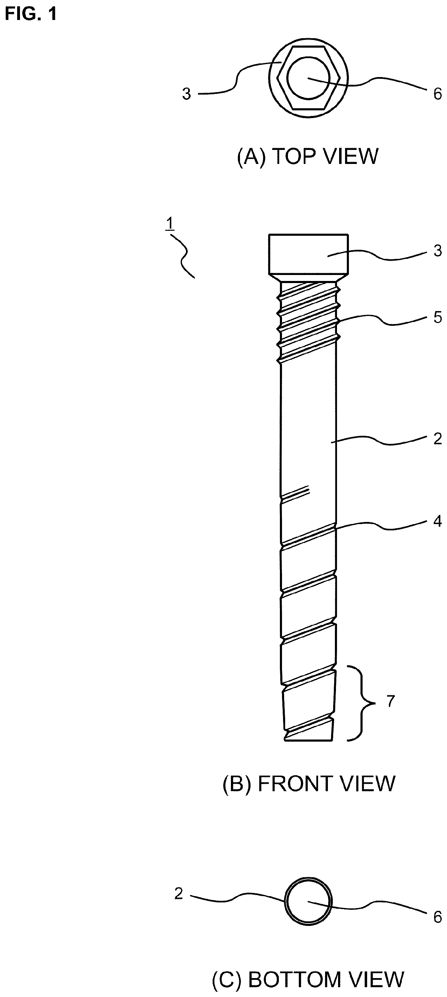

### [1] Internal fixation device with spiral grooves on shaft (FIG. 1)

A representative embodiment disclosed in this application is internal fixation device (1) to be inserted through a pedicle into bone cement filled into a vertebral body, the device comprising cylindrical shaft (2) and connection (3) at a distal end of the shaft that can be connected to a screwdriver. The shaft has helical grooves (4) on a proximal side.

This provides an internal fixation device that is inserted through the pedicle into bone cement pre-filled into the vertebral body, the device being easy-to-remove, but exhibiting internal fixation performance equivalent to that of a screw.

### [2] Groove is deeper at tip

In the internal fixation device of [1], the groove is deeper on the proximal side than on the distal side of the shaft.

This allows bone cement to smoothly enter groove (4) for secure fixation when internal fixation device (1) is screwed, while internal fixation device (1) can be removed with smaller force by applying rotation opposite to that of screwing.

### [3] Shaft is tapered at tip

In internal fixation device (1) of [1] or [2], the shaft has tapered section (7) at the proximal end that gradually decreases in diameter from the center.

This reduces the force required to screw in internal fixation device (1) and the force required to remove it.

### [4] Threads on distal side of shaft

In any one of internal fixation devices (1) of [1] to [3], the shaft has the grooves on the proximal side and projecting threads (5) on the distal side.

This ensures that threads (5) are securely fixed to the pedicle and that the bone cement is held stable.

### [5] Grooves and threads have the same lead

In internal fixation device (1) of [4], grooves (4) and threads (5) are formed to have the same lead.

As in[1], this allows bone cement to smoothly enter groove (4) for secure fixation when internal fixation device (1) is screwed, while internal fixation device (1) can be removed with smaller force by applying rotation opposite to that of screwing. Lead is the travel distance in the direction of the central axis per unit number of revolutions. By forming grooves (4) and threads (5) to have the same lead, resistance is distributed without strong resistance applied to one of grooves (4) and threads (5). The term "the same" herein is not intended to mean mathematically exactly the same, but allows for tolerance to the extent that moderate resistance is distributed.

### [6] The number of grooves is smaller than the number of threads

In internal fixation device (1) of [5], the number of the grooves is smaller than the number of ridges in the thread.

This allows for proper design of the bonding force between internal fixation device (1) and the bone cement while maintaining a secure fixation by threads (5) with the pedicle. As described in[5] above, it is suitable that grooves (4) and threads (5) are formed to have the same lead, which creates a new challenge of setting the appropriate contact area with the object, but that challenge is solved by designing the number of grooves and the number of the threads independently.

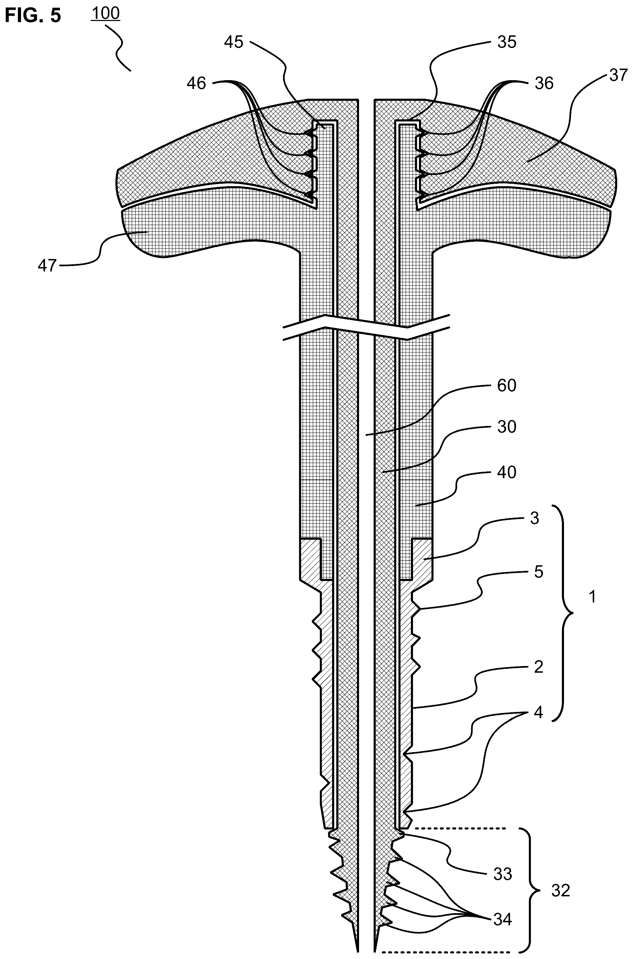

### [7] Treatment device for operating internal fixation device of the present invention (FIG. 5, FIG. 6 and FIG. 7)

A representative embodiment disclosed in this application is treatment device (100) which includes cylindrical screwdriver (40) connectable to internal fixation device (1) that is inserted through the pedicle into bone cement filled into the vertebral body, and inner cylinder (30) that can be inserted into the screwdriver. Internal fixation device (1) is configured as follows.

The internal fixation device is provided with cylindrical shaft (2) and connection (3) at a distal end of the shaft to be connectable to the screwdriver, and has through hole (6) that allows the inner cylinder to pass from the distal end to the proximal end along the central axis. The shaft has helical grooves (4) on a proximal side.

The screwdriver has a through hole through which the inner cylinder can pass from the head to the tip along the central axis. The screwdriver can also fit into connection (3) of the internal fixation device to connect to the internal fixation device when moved along the central axis, so that the force in the direction of rotation around the central axis can be transmitted to the internal fixation device

The inner cylinder has connection (35) that is inserted from the distal end of the screwdriver into the through hole of the screwdriver and connected to the screwdriver at the distal end, and tip section (32) that protrudes more proximally than the tip of the internal fixation device connected to the screwdriver. The inner cylinder has elastic tongue piece (31) with protrusion (33) at the tip section thereof.

When the inner cylinder is inserted into the screwdriver and the internal fixation device, the tongue piece can bend toward the central axis and move in the through hole of the screwdriver and the internal fixation device in the direction of the central axis. When the inner cylinder is inserted into the through hole of the screwdriver and connected to the screwdriver at the distal end, and also connected to the internal fixation device, the protrusion contacts the tip of the internal fixation device.

This provides treatment device (100) suitable for the operation of inserting internal fixation device (1) of [1] to [6] from the pedicle into the bone cement filled in the vertebral body. When screwdriver (40) with inner cylinder (30) inserted therein is connected to internal fixation device (1), they are integrated. This integration is achieved by connecting screwdriver (40) with internal fixation device (1) at connection (3) of the internal fixation device, and connecting the inner cylinder with the distal end of the screwdriver, and suppressing the inner cylinder by causing the protrusion of the tongue piece to contact the tip of the internal fixation device.

### [8] Fixation by insertion of guide pin

In treatment device (100) of [7], the inner cylinder has a through hole through which a guide pin (15, not shown FIGS. 5 to 7) can pass from the distal end to the proximal end along the central axis. When the inner cylinder is inserted into the through hole of screwdriver (40), connected at the distal end with the screwdriver, and also connected with internal fixation device (1), the guide pin is further inserted into the inner cylinder to thereby regulate the plurality of tongue pieces not to bend in the direction of the central axis, bringing the protrusion into contact with the tip section of the internal fixation device, and regulating the movement of the inner cylinder along the central axis back toward the through hole of the internal fixation device.

This makes the integration described in[7] securer.

### [9] Threads for tapping on tip section of inner cylinder

In treatment device (100) of [7] or [8], the inner cylinder is provided with threads (34) for tapping on an outer circumference of the tip section.

This allows the internal fixation device to reach the vertebral body smoothly even when the diameter of the bone tunnel formed in the pedicle is small when the integrated treatment device (100) described in[7] is operated to insert internal fixation device (1) from the pedicle into the vertebral body.

### [10] Internal fixation device has rounded proximal end

In treatment device (100) of [7] or [8], the internal fixation device has a rounded proximal end.

This reduces the risk of damaging the balloon when inserting balloon catheter (12) into through hole (6) of internal fixation device (1) and cause balloon catheter (12) pass through balloon (11).

### [11] Internal fixation device has grooves on proximal side and threads on distal side

In treatment device (100) of [7] or [8], the shaft of the internal fixation device has the grooves on the proximal side and projecting threads (5) on the distal side.

This ensures that threads (5) are securely fixed to the pedicle and that the bone cement is held stable, as in[4].

### [12] Grooves and threads have the same lead

In treatment device (100) of [11], the grooves in the internal fixation device and the threads are formed to have the same lead.

This allows bone cement to smoothly enter groove (4) for secure fixation when internal fixation device (1) is screwed, while internal fixation device (1) can be removed with smaller force by applying rotation opposite to that of screwing, as in[5].

### [13] The number of grooves is smaller than the number of threads

In treatment device (100) of [12], the number of the grooves in the internal fixation device is smaller than the number of ridges in the thread.

This allows for proper design of the bonding force between internal fixation device (1) and the bone cement while maintaining secure fixation by threads (5) with the pedicle, as in[6].

### 2. Details of the embodiments

Details of the embodiments will be described in more detail.

### [Embodiment 1]

FIG. 1 is a top view (a), a front view (b) and a bottom view (c) of an example configuration of an internal fixation device according to the present invention. Since the back view and left/right side views are the same as the front view (b), except that grooves 4 and threads 5 are placed at extended positions from the front view (b), illustration thereof are therefore omitted.

Internal fixation device 1 has a columnar shaft 2 and connection 3 that is connectable to a screwdriver at a distal end of shaft 2. Shaft 2 has helical grooves 4 on a proximal side. Suitably shaft 2 is cylindrical in shape as illustrated in the figures, and has through hole 6 that passes from the distal end to the proximal end along a central axis. Through hole 6 can, for example, allow a guide pin to pass through. As described below, through hole 6 may be configured to allow passage of a bone drilling tool, a balloon catheter, a bone cement injector, and/or the like. Connection 3 is configured as a hexagonal prismatic recess to engage with a screwdriver (not shown) and to engage with for example a hexagonal wrench as shown in the figures so as to rotate the entire internal fixation device 1.

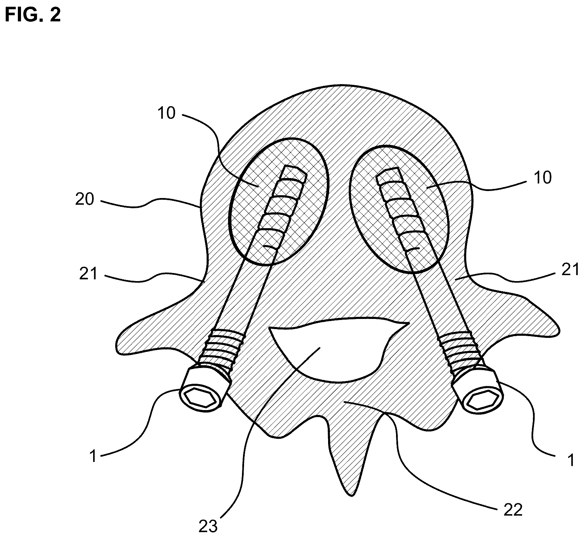

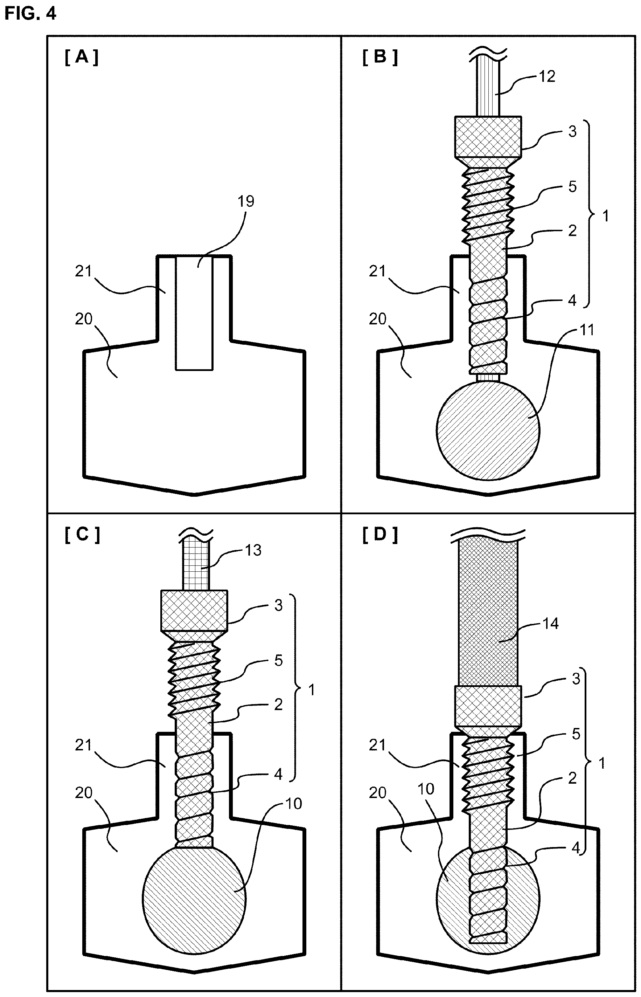

FIG. 2 is an explanatory drawing overviewing an example use of internal fixation device 1. In the patient's standing position, a plurality of vertebral bodies 20 stacked vertically form the spine, one of vertebral bodies 20 is shown in FIG. 2 as if seen in perspective from above and below. Vertebral body 20 is supported by a pair of pedicles 21 on each side, which are connected by vertebral arch 22. The area bounded by vertebral body 20, pedicles 21, and vertebral arch 22 is spinal canal 23. A compression fracture is a condition in which the vertebral body is compressed and crushed by forces in the vertical direction. In the treatment using internal fixation device 1 according to the present invention, as described below with reference to FIG. 4, a space is re-formed in vertebral body 20 and bone cement 10 is injected thereinto, and before it hardens, internal fixation device 1 is inserted (screwed) and supported from pedicle 21 side. Internal fixation device 1 is inserted through pedicle 21 into bone cement 10 pre-filled into vertebral body 20, and as bone cement 10 hardens, it is supported by internal fixation device 1 from pedicle 21 and fixed into vertebral body 20.

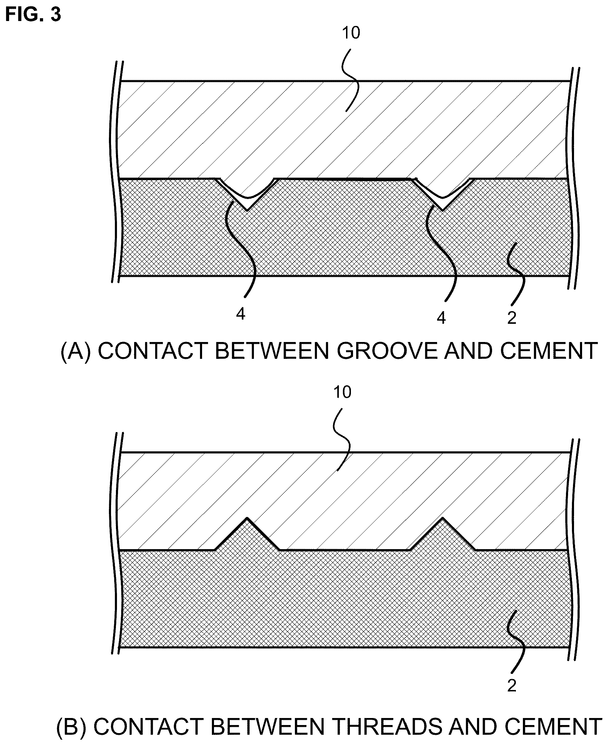

FIG. 3 is an explanatory drawing illustrating a solution principle of the present invention. The upper side (a) shows a cross-sectional view of the joint between bone cement 10 and shaft 2 provided with grooves 4, and the lower side (b) shows a cross-sectional view of the joint assuming that shaft 2 has threads instead of grooves 4. Since the threads here are different from threads 5 on the distal side of shaft 2, they will be described without the reference sign 5. Here, grooves 4 are formed to recess in the central axial direction from the outer circumference of shaft 2, and the threads are formed to protrude from the outer circumference of shaft 2. When shaft 2 has threads (b), the threads tap bone cement 10, i.e., the threads advance while pushing bone cement 10 around the protrusion of the threads when it is screwed into bone cement 10, the surface of the threads adhere to bone cement 10. When the threads tap bone cement 10, in addition to the driving force added to shaft 2, driving force from the rotational force of the tapping is also added. On the other hand, when shaft 2 has grooves 4 (a), grooves 4 have no tapping action on bone cement 10, so the penetration of bone cement 10 into grooves 4 is exclusively due to the viscosity of bone cement 10. Therefore, even with high viscosity, bone cement 10 does not always reach the bottom of grooves 4, and gaps may remain as illustrated in the figure. Thus, threads and grooves have very different actions on bone cement, resulting in very different adhesion. Forming grooves 4 on shaft 2 makes it easier to pull out shaft 2 compared to forming the threads, while making it harder to pull out shaft 2 compared to shaft 2 alone. Therefore, by appropriately adjusting the length, number, and depth of grooves 4, proper ease of removal can be achieved.

While grooves 4 illustrated in the figure is triangular with a pointed bottom, the shape is arbitrary and the angle of the bottom can be any angle; more acute or more obtuse than the right angle illustrated can be employed, and the bottom can be flat or arc-shaped. While the threads used for comparison have sharp tips, the same solution principle described above applies to smoothly chamfered threads.

While the depth of grooves 4 of internal fixation device 1 can be uniform, it is more suitable if it is deeper on the proximal (tip) side than on the distal (head) side of shaft 2. Since the proximal side (tip side) that initially contacts bone cement 10 is deeper, when internal fixation device 1 is screwed into bone cement 10 while rotating along the spiral of grooves 4, bone cement 10 enters deeper into grooves 4, and as it is screwed, is fed along the spiral of grooves 4 to the distal side (head side) where grooves 4 are shallower. So, bone cement 10 smoothly enters grooves 4 and is securely fixed. During removal, internal fixation device 1 is separated from bone cement 10 while rotating reversely along the spiral of grooves 4, so the force required for removal can be reduced.

More suitably, shaft 2 of internal fixation device 1 has tapered section 7 that gradually decreases in diameter from the center toward its proximal end. When internal fixation device 1 is screwed into bone cement 10, the resistance when first inserting it into bone cement 10 is reduced, and the force required to remove it is also reduced.

The distal side (head side) of shaft 2 of internal fixation device 1 should be provided with threads 5. Threads 5 is a spiral protrusion protruding outward from the outer surface of shaft 2 and functions to cause internal fixation device 1 (shaft 2) to advance along its central axis by tapping the surrounding bone as it rotates. Threads 5 advances by tapping pedicle 21 as illustrated in FIG. 2, securely fixing internal fixation device 1 and stabilizing bone cement 10 in vertebral body 20 into which shaft 2 has been inserted.

Threads 5 and grooves 4 are suitably formed to have the same lead. Since lead is the travel distance of shaft 2 along the central axis relative to the amount of rotation, it is desirable that the travel distance that shaft 2 advances while threads 5 taps pedicle 21 and the travel distance that shaft 2 advances along the helix of grooves 4 when it is screwed into bone cement 10 should match.

However, the travel distances do not necessarily need to match precisely, and can be intentionally made different. For example, by setting the lead of grooves 4 larger than the lead of threads 5, grooves 4 at the tip of shaft 2 is partially screwed into bone cement 10, and after threads 5 begins tapping the pedicle, the force can be exerted to bring bone cement 10 toward the pedicle as internal fixation device 1 (shaft 2) is rotated.

The number grooves 4 can be different from the number of ridges in threads 5. For example, the number of grooves 4 should be smaller than the number of ridges in threads 5. This allows for proper design of the bonding force between internal fixation device 1 and bone cement 10 while maintaining a secure fixation by threads 5 with pedicle 21. As described above, it is suitable that grooves 4 and threads 5 are formed to have the same lead, which creates a new challenge of setting the appropriate contact area with the object, but that challenge is solved by designing the number of grooves and the number of the threads independently. Reducing the number of grooves 4 reduces the area of contact with bone cement 10, thereby reducing the force required for pulling out. On the other hand, by increasing the contact area between pedicle 21 and threads 5, the fixation of internal fixation device 1 to pedicle 21 can be strengthened. By optimizing the number of grooves and/or the threads rather than the pitch, the relationship between bone cement 10 and grooves 4 and the relationship between pedicle 21 and threads 5 can be optimized independently.

FIG. 4 is an explanatory drawing showing a procedure of an example use of internal fixation device 1 according to the present invention. As shown in FIG. 2, the spine, in the patient's standing position, consists of a plurality of vertically stacked vertebral bodies 20, each of which is supported by a pair of pedicles 21 on each side, the pedicles 21 being connected by the vertebral arch 22. FIG. 4 shows a cross-section from the patient's body surface (not shown) through one pedicle 21 to vertebral body 20, assuming the body surface as the upper side.

Step A: Incise the skin on the side that allows access to the affected vertebral body 20, and form bone tunnel 19 leading to vertebral body 20 in pedicle 21. The diameter of bone tunnel 19 may be just large enough for shaft 2 of internal fixation device 1 to be inserted or a bone tunnel into which the guide pin described below can be inserted, and the length is generally long enough to reach vertebral body 20.

Step B: Insert shaft 2 of internal fixation device 1 into the formed bone tunnel 19. A guide pin (not shown) should be inserted into vertebral body 20 through bone tunnel 19 from outside the body, and internal fixation device 1 is inserted along the guide pin. A section of shaft 2 provided no protrusion such as threads 5 should be inserted into bone tunnel 19, and the section of threads 5 may desirably stop outside of pedicle 21. Balloon catheter 12 is inserted into through hole 6 of shaft 2 of internal fixation device 1, its tip section is allowed to reach into vertebral body 20, and balloon 11 is inflated at the tip to create space inside vertebral body 20. At this time, screwdriver 14 (not shown) may be connected to the connection of head 3 of internal fixation device 1 and threads 5 may be slightly pre-torqued into pedicle 21. Prior to insertion of balloon catheter 12, a puncture needle or bone drilling device may be introduced from outside the body into vertebral body 20 through bone tunnel 19 to perform bone drilling or other procedures from inside vertebral body 20. It is more suitable if the tip of internal fixation device 1 is smoothly rounded. As described above, in this step B in which balloon 11 is introduced into vertebral body 20 through through hole 6 of internal fixation device 1 and taken out after it is inflated, the risk of damage to balloon 11 from touching the tip of internal fixation device 1 can be lowered.

Step C: Insert cement injector 13 instead of balloon catheter 12 into through hole 6 of shaft 2 of internal fixation device 1, allow the tip of cement injector 13 to reach into vertebral body 20, and inject bone cement 10 from the tip section of cement injector 13 into vertebral body 20. Here, screwdriver 14 (not shown in[c]) may be connected in advance to the connection of head 3 of internal fixation device 1, so that cement injector 13 can be introduced to the affected vertebral body 20 through the through hole provided along the central axis of screwdriver 14 and communicating with through hole 6 of the connected internal fixation device 1 as described above.

Step D: Connect screwdriver 14 to the connection of head 3 of internal fixation device 1 and screw threads 5 into pedicle 21. In the tip (proximal end) of internal fixation device 1, a portion of shaft 2 in which grooves 4 is formed is screwed into bone cement 10.

Screwdriver 14 is removed from internal fixation device 1, and the opening is sutured to complete the procedure.

Screwdriver 14 is not shown in[B] and[C] in FIG. 4.However, as mentioned above, it is suitable that screwdriver 14 also has a through hole communicating with through hole 6 of internal fixation device 1, so that steps B and C can be proceeded with screwdriver 14 attached to internal fixation device 1 from the beginning of step B. By sequentially introducing a puncture needle, a bone drilling device (not shown), balloon catheter 12, and cement injector 13 into vertebral body 20 through a through hole leading from screwdriver 14 to internal fixation device 1, the drilling of the bone in vertebral body 20, expansion of the space using a balloon, and filling of bone cement 10 can be performed with the initially installed screwdriver 14 attached. This reduces the number of procedures to change instruments, shortening the operation time, and also minimizes the time between filling bone cement 10 and screwing in internal fixation device 1, allowing internal fixation device 1 to be screwed in with time to spare before bone cement 10 hardens.

### [Embodiment 2]

Internal fixation device 1 according to the present invention is more suitable when used in combination with treatment device 100 according to the aforementioned international patent application (PCT/JP2022/036147).

FIG. 5 is an explanatory drawing illustrating Embodiment 2 as an example use of internal fixation device 1. In FIG. 5, the scale in the direction of the central axis (vertical direction on the paper) is compressed, and the direction along the patient's body surface (horizontal direction on the paper) is emphasized. Treatment device 100 includes a cylindrical screwdriver 40 that can screw internal fixation device 1 into pedicle 21, and inner cylinder 30 that is inserted into screwdriver 40, and is configured as follows.

The tip of internal fixation device 1 is rounded to allow soft vertebral body drilling devices such as balloons to be inserted and removed, and through hole 6 is of a diameter that allows inner cylinder 30 to pass from head 3 to the tip along the central axis.

Screwdriver 40 is configured to fit into the connection of head 3 of internal fixation device 1 to connect to internal fixation device 1 when moved along the central axis, so that the force in the direction of rotation around the central axis can be transmitted to internal fixation device 1. For example, the tip of screwdriver 40 should have a hexagonal wrench shape (hexagonal prism) and the connection of head 3 of internal fixation device 1 may desirably have a hexagonal groove (recess). The shape of the tip of screwdriver 40 and the shape of the connection of head 3 of internal fixation device 1 can be a shape other than hexagonal, such as star-shape, as long as engagement is achieved upon insertion. Sliding screwdriver 40 along the central axis into the connection of head 3 of internal fixation device 1 allows the tip of screwdriver 40 to fit into the connection of head 3 of internal fixation device 1, allowing screw-in of internal fixation device 1 via screwdriver 40 and easy removal by pulling out screwdriver 40.

Inner cylinder 30 has tip section 32 that is inserted from the distal end of screwdriver 40 through the through holes of screwdriver 40 and internal fixation device 1 and protrudes proximally than the tip of internal fixation device 1. Inner cylinder 30 is connected integrally to screwdriver 40 and internal fixation device 1 with tip section 32 protruding proximally than the tip of internal fixation device 1.

This provides a treatment device suitable for the techniques of artificial disc replacement, spinal fusion, vertebroplasty, and pedicle formation for spinal disorders.

An example structure for integrating internal fixation device 1, screwdriver 40, and inner cylinder 30 will be described.

As shown in FIG. 5, screwdriver 40 and inner cylinder 30 are configured to be connected at the distal end. The distal end of screwdriver 40 is provided with a cylindrical connection 45 with external threads 46 on its outer circumference, and the distal end of inner cylinder 30 has a groove-shaped connection 35 that can accommodate the cylinder and is provided with internal threads 36 that engage with the external threads 46. The relationship between recess and protrusion, and external threads and internal threads may be reversed, or other connection mechanisms may be used. As illustrated in FIG. 5, screwdriver 40 has handle 47, and inner cylinder 30 has handle 37, these handles being preferably integrated for easy gripping when connected. In addition to threading mechanism as in this example, a latch mechanism (not shown) may be provided to prevent removal of the connection.

As shown in FIG. 6, the tip (proximal end) of inner cylinder 30 is divided into a plurality of tongue pieces 31 in the direction along the central axis, and each of the plurality of tongue pieces 31 has protrusion 33 in the direction away from the central axis. Protrusion 33 has the function of a notch that regulates internal fixation device 1 from being pulled out of inner cylinder 30, as described below, so the reference sign 33 is referred to as a notch. The plurality of tongue pieces 31 are formed, for example, by dividing cylindrical inner cylinder 30 with slits from the tip side. The number of divisions can be arbitrarily set to, for example, 4, 6, 2, or 3. In addition, some processing (e.g., heat treatment, reduction in thickness, laminating highly elastic metal materials) may be applied to provide adequate elasticity. Elasticity of tongue piece 31 is designed to be moderate. For example, elasticity is designed such that when inner cylinder 30 is passing through the through hole in screwdriver 40, notch 33 is pressed against the inner wall of the through hole to flex in the central axial direction to allow the passage of inner cylinder 30, and when notch 33 exits proximal to the tip of internal fixation device 1, the flexure is restored and notch 33 is caught on the tip of internal fixation device 1. When notch 33 is smooth in shape, it can be configured so that even after notch 33 exits the tip of internal fixation device 1, the force to pull inner cylinder 30 can still pull notch 33 into through hole 6 of internal fixation device 1 and inner cylinder 30 can be pulled out by flexing tongue piece 31 in the central axis direction again.

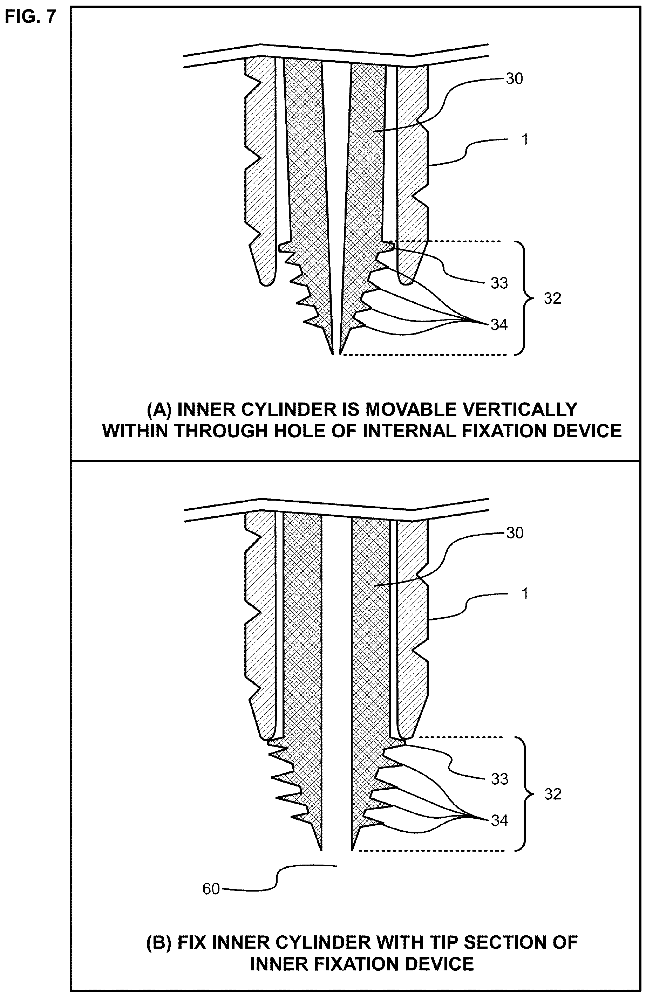

FIG. 7 is an explanatory diagram schematically illustrating a cross-sectional structure to show an example configuration of tip section 32 of inner cylinder 30. As shown in upper side (a), inner cylinder 30 can move within the through hole in the central axial direction by bending tongue piece 31 described above toward the central axis when inner cylinder 30 is inserted into the connected screwdriver 40 and internal fixation device 1. As shown in lower side (b), when inner cylinder 30 is inserted into the connected screwdriver 40 and internal fixation device 1 and is connected at the distal end with screwdriver 40 as described above, tip section 32 protrudes more proximally than the tip of internal fixation device 1 and the flexure of tongue piece 31 returns to cause notch 33 to contact the tip of internal fixation device 1. In this state, when guide pin 60 is inserted into inner cylinder 30, tongue piece 31 cannot bend in the direction of the central axis when inner cylinder 30 is to be pulled out, notch 33 protrudes outward from the diameter of through hole 6 of internal fixation device 1, and the movement of inner cylinder 30 back along the central axis in the distal direction, i.e., toward through hole 6 of internal fixation device 1 is restricted. This provides securer integration. In simple terms, when internal fixation device 1 is to be pulled out of inner cylinder 30, notch 33 contacts the tip of internal fixation device 1 for the restriction of not to be pulled out. This makes the integration securer.

More suitably tip section 32 of inner cylinder 30 is provided with threads 34 for tapping on the outer surface thereof. The threads 34 should be formed in such positions that when internal fixation device 1, screwdriver 40 and inner cylinder 30 are integrated as described above, they smoothly connect with the tapered section of internal fixation device 1.

The following describes a treatment method for artificial disc replacement, spinal fusion, vertebroplasty, and pedicle formation for spinal disorders using treatment device 100 according to the present invention.

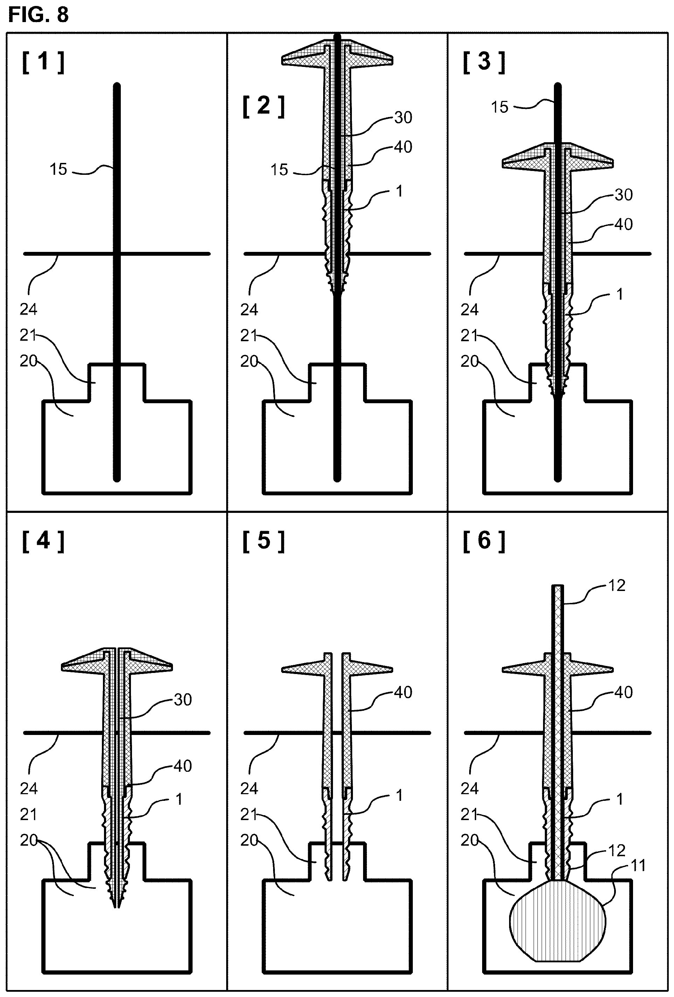

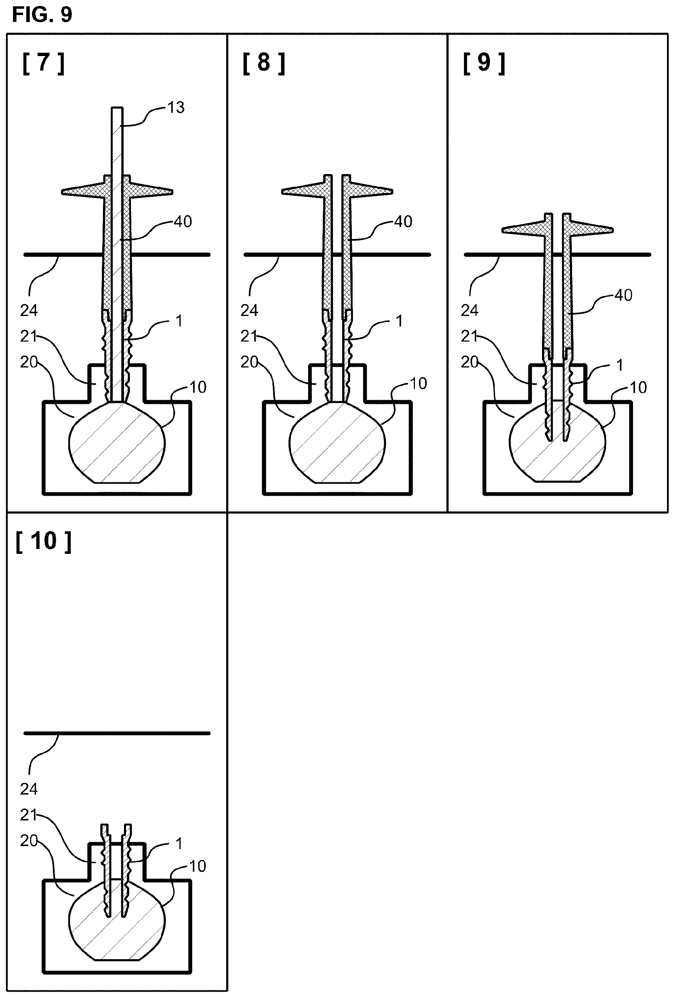

FIGS. 8 and 9 are explanatory drawings schematically illustrating a treatment procedure using treatment device 100 of Embodiment 2; FIG. 8 shows the first half and FIG. 9 shows the second half. Although FIGS. 8 and 9 illustrate vertebroplasty, the same method of inserting internal fixation device 1 into the vertebral body is also used for vertebral augmentation in spinal fusion and artificial disc replacement (not shown). FIGS. 8 and 9 schematically show a cross-section of the patient's affected area from the outer skin 24 through pedicle 21 to vertebral body 20, with the upper side of the paper as distal side and the lower side as proximal side. The scale in the direction of the central axis (vertical direction on the paper) is compressed, and the direction along the patient's body surface (horizontal direction on the paper) is emphasized. Since pedicles 21 are on each side of the vertebral bodies 20, the same procedure is performed from the left and right for one vertebral body 20, FIGS. 8 and 9 show one of them.

Step 1 ([1]): Incise and drill subcutaneous tissue from the patient's back body surface, i.e., skin 24, to the affected vertebral body 20, and introduce guide pin 15 through pedicle 21 to vertebral body 20.

Step 2 ([2]): Connect internal fixation device 1 and screwdriver 40, insert inner cylinder 30 into an inner through hole of internal fixation device 1, combine inner cylinder 30 with the tip protruding from the tip section of internal fixation device 1, and insert inner cylinder 30 into the patient's body along guide pin 15, covering guide pin 15.

Step 3 ([3]): Push connected internal fixation device 1, screwdriver 40, and inner cylinder 30 further proximally along guide pin 15, and screw tips of inner cylinder 30 and internal fixation device 1 into pedicle 21.

Step 4 ([4]): After screwing the tip of internal fixation device 1 until it reaches near the entrance of vertebral body 20, remove guide pin 15. At this time, notch 33 of inner cylinder 30 is in contact with the tip of internal fixation device 1 to deter inner cylinder 30 from spontaneously pulling out, but guide pin 15 has been pulled out, and the force to pull out inner cylinder 30 allows tongue piece 31 to easily flex in the central axis direction to pull out inner cylinder 30.

Step 5 ([5]): Remove inner cylinder 30 At this time, screwdriver 40 is simply inserted into head 3 of internal fixation device 1, and is held down so that it cannot be pulled out. Although not specifically illustrated, this step may introduce vertebral drilling device to drill within vertebral body 20 to fill cement for reinforcement through the through holes of screwdriver 40 and internal fixation device 1, if necessary.

Step 6 ([6]): Introduce balloon catheter 12 into vertebral body 20 through the through holes of screwdriver 40 and internal fixation device 1, and inflate balloon 11 in vertebral body 20. For example, balloon 11 is inflated by injecting a contrast medium through balloon catheter 12 and applying pressure. The purpose is to restore vertebral bodies 20 to their original size when vertebral bodies 20 are compression fractured due to osteoporosis or other causes.

Step 7 ([7]): Pull balloon catheter 12 out of the through holes of screwdriver 40 and internal fixation device 1 and insert cement injector 13 instead to inject bone cement 10 into vertebral body 20. Since the volume of cement that can be filled at one time by cement injector 13 is about 1.5 ml, in the case of vertebroplasty for a fracture, for example, about 10 ml of bone cement 10 is filled into vertebral body 20 by repeatedly delivering cement 6 to 7 times in total, about 3 times from each side. When applied to vertebral body augmentation procedures other than vertebroplasty, spinal fusion, and artificial disc replacement, the amount of cement to fill should be adjusted according to the bone condition.

Step 8 ([8]): Pull cement injector 13 out through the through holes of screwdriver 40 and internal fixation device 1.

Step 9 ([9]): Screw internal fixation device 1 into the filled bone cement 10 using screwdriver 40.

Step 10 ([10]): Pull screwdriver 40 out and suture incised skin 24 to complete the treatment.

As described above, treatment device 100 allows internal fixation device 1 to be screwed into the filled bone cement 10 immediately after bone cement 10 is filled, is suitable for spinal fusion, vertebral body augmentation in artificial disc replacement, vertebroplasty, and pedicle formation.

The filled bone cement 10 is soft enough that internal fixation device 1 can be screwed in without tapping using threads 34 of tip section 32 of inner cylinder 30. Similarly, internal fixation device 1 can be screwed into pedicle 21 without tapping using tip section 32 of inner cylinder 30.

Treatment device 100 is also suitable for removal of internal fixation device 1. After the affected area is incised and drilled to the screw head, guide pin 15 is inserted into internal fixation device 1, screwdriver 40 is introduced along it to internal fixation device 1, and the tip is pushed in; this can simply connect screwdriver 40. Internal fixation device 1 can then be pulled out by rotating screwdriver 40.

While the present invention made by the present inventor has been described in detail with respect to the embodiments thereof, the present invention is not limited to these embodiments. Various changes may be made without departing from the spirit and scope of the present invention.

### Industrial Applicability

The present invention relates to a columnar internal fixation device for medical use, and is particularly suitable for use in internal fixation surgery of affected vertebral body.

### EXPLANATION OF SIGN

1 Internal fixation device
2 Shaft
3 Head (screwdriver connection)
4 Groove
5 Ridge
6 Through hole
7 Tapered portion
10 Bone cement
11 Balloon
12 Balloon catheter
13 Cement injector
14 Screwdriver
15 Guide pin
19 Bone tunnel
20 Vertebral body
21 Pedicle
22 Vertebral arch
23 Spinal canal
24 Skin
30 Inner cylinder
31 Tongue piece of inner cylinder
32 Tip section of inner cylinder
33 Notch
34 Thread at tip section of inner cylinder
35 Connection with screwdriver
36 Internal thread
37 Handle of inner cylinder
40 Screwdriver
45 Connection with inner cylinder
46 External thread
47 Handle of screwdriver
100 Treatment device

## Claims

1. An internal fixation device to be inserted through a pedicle into bone cement filled into a vertebral body, the device comprising
a cylindrical shaft and a connection at a distal end of the shaft that can be connected to a screwdriver,
wherein the shaft has helical grooves on a proximal side.

2. The internal fixation device according to claim 1, wherein the groove is deeper on the proximal side than on the distal side of the shaft.

3. The internal fixation device according to claim 1 or 2, wherein the shaft has a tapered section at the proximal end that gradually decreases in diameter from the center.

4. The internal fixation device according to any one of claims 1 to 3, wherein the shaft has the grooves on the proximal side and projecting threads on the distal side.

5. The internal fixation device according to claim 4, wherein the grooves and threads are formed to have the same lead.

6. The internal fixation device according to claim 5, wherein the number of the grooves is smaller than the number of ridges in the thread.

7. A treatment device that includes a cylindrical screwdriver connectable to internal fixation device that is inserted through a pedicle into bone cement filled into a vertebral body, and an inner cylinder that can be inserted into the screwdriver, wherein
the internal fixation device is provided with a cylindrical shaft, and a connection at a distal end of the shaft to be connectable to the screwdriver, and has a through hole that allows the inner cylinder to pass from a distal end to a proximal end along a central axis, wherein the shaft has helical grooves on a proximal side;
the screwdriver has a through hole through which the inner cylinder can pass from the head to the tip along the central axis, the screwdriver can be fit into the connection of the internal fixation device to connect to the internal fixation device when moved along the central axis, so that the force in the direction of rotation around the central axis can be transmitted to the internal fixation device;
the inner cylinder has a connection that is inserted from the distal end of the screwdriver into the through hole and connected to the screwdriver at the distal end, and a tip section that protrudes more proximally than the tip of the internal fixation device connected to the screwdriver;
the inner cylinder has an elastic tongue piece with a protrusion at a tip section thereof;
when the inner cylinder is inserted into the screwdriver and the internal fixation device, the tongue piece can bend toward the central axis and move in the through hole of the screwdriver and the internal fixation device in the direction of the central axis; and
when the inner cylinder is inserted into the through hole of the screwdriver and connected to the screwdriver at the distal end, and also connected to the internal fixation device, the protrusion contacts the tip of the internal fixation device.

8. The treatment device according to claim 7, wherein
the inner cylinder has a through hole through which a guide pin can pass from the distal end to the proximal end along the central axis; and
when the inner cylinder is inserted into the through hole of screwdriver, connected at the distal end with the screwdriver, and also connected with internal fixation device, the guide pin is inserted into the inner cylinder to thereby regulate the plurality of tongue pieces not to bend in the direction of the central axis, bringing the protrusion into contact with the tip section of the internal fixation device, and regulating the movement of the inner cylinder along the central axis back toward the through hole of the internal fixation device.

9. The treatment device according to claim 7 or 8, wherein the inner cylinder is provided with threads for tapping on an outer circumference of the tip section.

10. The treatment device according to claim 7 or 8, wherein the internal fixation device has a rounded proximal end.

11. The treatment device according to claim 7 or 8, wherein the shaft of the internal fixation device has the grooves on the proximal side and projecting threads on the distal side.

12. The treatment device according to claim 11, wherein the grooves in the internal fixation device and the threads are formed to have the same lead.

13. The treatment device according to claim 12, wherein the number of the grooves in the internal fixation device is smaller than the number of ridges in the thread.
